(19) Europäisches Patentamt
European Patent Office
Office européen des brevets

(11) Veröffentlichungsnummer: **0 175 896**
**B1**

# (12) EUROPÄISCHE PATENTSCHRIFT

(45) Veröffentlichungstag der Patentschrift: **19.07.89**

(51) Int. Cl.$^4$: **C 07 C 85/06**, C 07 C 85/08, C 07 C 87/123

(21) Anmeldenummer: **85109873.1**

(22) Anmeldetag: **06.08.85**

## (54) Verfahren zur Herstellung von Aminen.

(30) Priorität: **31.08.84 DE 3432015**

(43) Veröffentlichungstag der Anmeldung: **02.04.86 Patentblatt 86/14**

(45) Bekanntmachung des Hinweises auf die Patenterteilung: **19.07.89 Patentblatt 89/29**

(84) Benannte Vertragsstaaten: **AT BE CH DE FR GB IT LI LU NL SE**

(56) Entgegenhaltungen:
**DE-A- 3 116 395**
**FR-A- 2 370 030**
**US-A- 4 229 374**
**US-A- 4 409 399**
**Millmaster Onyx Group Inc**

(73) Patentinhaber: **SCHERING AKTIENGESELLSCHAFT**
**Berlin und Bergkamen**
**Waldstrasse 14 Postfach 15 40**
**D-4709 Bergkamen (DE)**

(72) Erfinder: **Hubert, Hans-Jürgen, Dr.**
**Weissdornweg 4**
**D-4700 Hamm 1 (DE)**
Erfinder: **Tillmetz, Klaus Dieter, Dr.**
**Olfener Landweg 4**
**D-4716 Olfen-Vinnum (DE)**

**EP 0 175 896 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Aminen durch Umsetzung von langkettigen Alkoholen oder Aldehyden mit primären Aminen, sekundären Aminen mit gleichen oder verschiedenen Substituenten oder Ammoniak bei erhöhter Temperatur und gegebenenfalls unter Druck in der Flüssigphase in reduzierender Atmosphäre in Gegenwart eines trägerfreien Katalysators.

Zahlreiche für dieses Verfahren geeignete Katalysatorsysteme sind beschrieben, beispielsweise in US-PS 4 409 399, in der der einschlägige Stand der Technik zur Flüssigphasen-Aminierung unter Anwendung von Trägersystemen bzw. trägerfreien Katalysatoren umfassend dargestellt ist. Hierzu gehört beispielsweise auch die DE-A-31 16 395, gemäß der ein Träger-Katalysatorsystem auf Kupfer/Nickel-Basis vorgeschlagen wird. Gemäß FR-A-2 370 030 sollen Metallkatalysatorsysteme, beispielsweise Kupfer/Nickel, durch Zugabe von Zink in ihrer Selektivität und Stabilität verbessert werden, während dies gemäß US-A-4 229 374 bei einem Kupfer/Zinn-Trägerkatalysator durch Alkalimetall-Zusatz bewirkt werden soll. Flüssigphasenaminierung bedeutet, daß der Alkohol oder der Aldehyd sich in der flüssigen Phase befindet, während Ammoniak oder die primären bzw. sekundären Amine sich bei den angegebenen Reaktionsbedingungen entweder in einer flüssigen oder Gasphase befinden.

Es wird festgestellt, daß alle zum Stand der Technik genannten Katalysatorsysteme für Aminierungsverfahren in der Flüssigphase eine verhältnismäßig geringe Aktivität aufweisen und daher in größeren Mengen bei hohen Drücken und Temperaturen einzusetzen sind. Eine verbesserte Aktivität in der Flüssigphasen-Aminierung zeigt nach US-PS 4 409 399 ein trägerfreies Katalysatorsystem auf der Basis von Kupfer- und Nickeloxid bzw. -hydroxid.

Dieses System weist gegenüber dem Stand der Technik den Vorteil einer verbesserten Filtrierbarkeit auf und ist daher leichter aus dem Reaktionsgemisch zu entfernen. Es kann jedoch nicht vermieden werden, daß gewisse Mengen Katalysator in gelöster Form in dem Reaktionsprodukt verbleiben, so daß sie — da sie weitere Umsetzungsreaktionen beeinträchtigen oder in gewissen Endprodukten nicht toleriert werden können — durch Destillation der Aminkomponente abgetrennt werden müssen.

Für die Destillation insbesondere langkettiger Amine wie z. B. Di-($C_{18}$-Alkyl)-methylamin sind — soweit sie sich überhaupt noch ohne Zersetzung destillieren lassen — einmal aufwendige Apparaturen notwendig, zum anderen bedeutet sie einen kosten-, zeit- und energieintensiven Verfahrensaufwand und verschlechtert letztlich die Ausbeute z. T. in erheblichem Maße. Für andere Reinigungsverfahren, wie Filtration mit Filtrierhilfsmitteln und Lösungsmitteln oder Extraktion, gilt sinngemäß das gleiche.

In der DE-OS 28 44 984 wird ein Katalysator aus einer Cu/Sn-Kombination auf einem porösen Träger vorgeschlagen. Vor Verwendung muß ein Aktivierungsprozeß in Wasserstoff oder Ammoniak bei Temperaturen von 250-600 °C über einen Zeitraum bis zu 24 Stunden durchgeführt werden.

Bei der Herstellung der Mono- und Dialkylierungsprodukte aus insbesondere primären Aminen entsprechen jedoch Alkoholumwandlungsgrad und Selektivität sowie die unter den angegebenen Aktivierungsbedingungen erreichbaren Aktivitäten der Katalysatoren noch nicht den Anforderungen der Praxis. Eine Destillation der Reaktionsprodukte ist auch hier noch erforderlich.

Aufgabe der vorliegenden Erfindung war es, einen Katalysator zu entwickeln, welcher die Nachteile des Standes der Technik überwindet und neben Unlöslichkeit in den Reaktionsprodukten eine verbesserte Aktivität, einen erhöhten Alkoholumwandlungsgrad und eine gesteigerte Selektivität, bezogen auf die verschiedenen Alkylierungsstufen, insbesondere aber auf tertiäre Amine (Dialkylierungsstufe) — auch bei primären Ausgangsaminen — aufweist.

Diese Aufgabe wird erfindungsgemäß gelöst durch ein Verfahren zur Herstellung von Aminen unter Verwendung von trägerfreien Katalysatoren aus Kupfer- und Zinnverbindungen, welche durch metallorganische Verbindungen aktiviert werden.

Gegenstand der Erfindung ist daher ein Verfahren zur Herstellung von Aminen durch Umsetzung von Alkoholen und/oder Aldehyden mit bis zu 26 Kohlenstoffatomen mit primären und/oder sekundären Aminen mit 1 bis 26 Kohlenstoffatomen oder Ammoniak in Gegenwart eines Katalysators bei erhöhter Temperatur und gegebenenfalls unter erhöhtem Druck in der Flüssigphase, das dadurch gekennzeichnet ist, daß als Katalysator ein trägerfreier Katalysator aus einer Kombination von Kupfer- und Zinnverbindungen verwendet wird, der vorzugsweise nach Voraktivierung eingesetzt wird, wobei zur Aktivierung insbesondere metallorganische Reduktionsmittel eingesetzt werden.

Als Alkohole und/oder Aldehyde für das erfindungsgemäße Verfahren kommen Verbindungen der allgemeinen Formeln

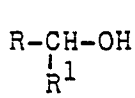 und/oder 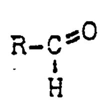

in Betracht, in welchen R und $R^1$ gleich oder verschieden sein können und Wasserstoff, lineare oder verzweigte, gesättigte oder ungesättigte, gegebenenfalls unter Aminierungsbedingungen inerte Heteroatome enthaltende aliphatische oder aromatische Reste mit zusammen bis zu 25 Kohlenstoffatomen, insbesondere 5 bis 21 und vorzugsweise 9 bis 17 Kohlenstoffatomen, bedeuten und worin R und $R^1$

zusammen mit dem Kohlenstoffatom einen gegebenenfalls substituierten Ring, vorzugsweise einen fünf- oder sechsgliedrigen Ring bilden können. Es können auch Mischungen der Alkohole und Aldehyde miteinander und/oder untereinander verwendet werden.

Erfindungsgemäß bevorzugt werden primäre Alkohole oder deren Mischungen, in denen $R^1 = H$, R ein aliphatischer Kohlenwasserstoffrest ist, der gegebenenfalls verzweigt sein kann und 9 bis 17 Kohlenstoffatome in der Kette enthält.

Als Beispiele für geeignete Alkohole seien genannt : Hexanol, Oktanol, Nonatol, Dekanol, Dodekanol, Hexadekanol, Oktadekanol, 2-Ethylhexanol, Oleylalkohol, Cerylalkohol, Cyclopentanol, Cyclohexanol, Cyclooktanol, Cyclodekanol, Furfuryl-(2)-alkohol, Benzylalkohol, Phenyläthylalkohol, die Oxyalkylierungsprodukte der Alkohole, insbesondere mit Äthylenoxyd und/oder Propylenoxid, die techn. Mischungen aus $C_{12}$-$C_{15}$-Alkoholen mit einer Linearität von 40-80 %.

Erfindungsgemäß bevorzugt werden geradkettige primäre Alkohole wie Oktanol, Dekanol, Dodekanol, Hexadekanol, Oktadekanol, Oleylalkohol.

Anstelle der genannten Alkohole können auch die korrespondierenden Aldehyde verwendet oder mitverwendet werden. Bevorzugt werden die korrespondierenden Aldehyde der erfindungsgemäß bevorzugt verwendeten Alkohole.

Die erfindungsgemäß mitverwendeten Amine sind Verbindungen der allgemeinen Formel

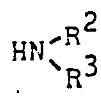

in welcher $R^2$ und $R^3$ gleich oder verschieden sein können und Wasserstoff, lineare oder verzweigte, gegebenenfalls unter Aminierungsbedingungen inerte Heteroatome enthaltende aliphatische oder araliphatische gesättigte oder ungesättigte Reste mit 1 bis 26 Kohlenstoffatomen, insbesondere 1-4 und vorzugsweise 1-2 Kohlenstoffatomen sein können und in welcher $R^2$ und $R^3$ zusammen mit dem Stickstoffatom einen Ring, vorzugsweise einen fünf- oder sechsgliedrigen Ring bilden können, der gegebenenfalls substituiert sein oder weitere Heteroatome enthalten kann. Geeignete Amine sind beispielsweise Monomethylamin, Dimethylamin, Monoethylamin, Diethylamin, Methylethylamin, Methylbutylamin, Piperidin, Morpholin, Pyrrolidin, 2-Ethylhexylamin, Dodecylamin, Hexadecylamin, die aus Fettsäuren mit 8-26 Kohlenstoffatomen hergestellten Amine. Erfindungsgemäß bevorzugt werden Monomethylamin und Dimethylamin. Die genannten Amine können allein oder als Mischung eingesetzt werden.

Das trägerfreie Katalysatorsystem für das erfindungsgemäße Verfahren ist entweder eine physikalische Mischung der jeweiligen Salze oder vorzugsweise eine gemeinsame Ausfällung aus einer wässrigen Salzlösung durch Zugabe von geeigneten Basen wie Natrium- oder Kaliumhydroxid bzw. -carbonat bis zum erforderlichen pH-Wert. Nach Abfiltration können die Metallhydroxide oder -oxide in üblicher Weise weiterbehandelt werden, wie waschen mit Wasser mit anschließender Ofen- oder Sprühtrocknung bei Temperaturen von — je nach Methode — 50 bis 500 °C über einen Zeitraum von einer bis 24 Stunden.

Das Katalysatorsystem enthält Kupfer- und Zinnverbindungen, vorzugsweise die entsprechenden Oxide und/oder Hydroxide in molaren Kupfer : Zinnverhältnissen von 0,1 bis 10, vorzugsweise von 1,5 bis 5,5.

Vor der Verwendung des Katalysators wird er vorteilhaft insbesondere mit metallorganischen Verbindungen in einem inerten Lösungsmittel, wie insbesondere aliphatische, cycloaliphatische oder aromatische Kohlenwasserstoffe mit Siedepunkten zwischen ca. 20 °C bis 200 °C, bei gegebenenfalls erhöhter Temperatur aktiviert.

Als Aktivatoren kommen grundsätzlich alle reduzierenden metallorganischen Verbindungen infrage, insbesondere aluminiumorganische und magnesiumorganische Verbindungen der allgemeinen Formeln

$$Al\ R^1 R^2 R^3$$
$$Mg\ R^1 R^2$$

mit $R^1$ und $R^2$ = geradkettiger oder verzweigter Alkylrest mit 1-12 C-Atomen und $R^3 = H$ oder $R^1$, $R^2$.

Als geeignete metallorganische Verbindungen können erfindungsgemäß z. B. mitverwendet werden : Triäthylaluminium, Diäthylaluminiumäthoxylat, Trimethylaluminium, Di-i-octylaluminiumhydrid, Tri-n-dodecylaluminium, Methyl-butylmagnesium, Di-n-octylmagnesium, insbesondere Diisobutylaluminiumhydrid, Butyloctylmagnesium, Triisobutylaluminium.

Diese Verbindungen werden allein oder in Mischung in mindestens äquivalenten Mengen, vorzugsweise in geringem Überschuß von 0,1-0,2 Äquivalenten, bezogen auf reduzierbare Gruppen im Katalysator, eingesetzt.

Andere Aktivierungsverfahren, z. B. Erhitzen in einer reduzierenden Atmosphäre, wie sie gemäß dem Stand der Technik üblicherweise angewandt werden, führen nicht zu den gewünschten, für das erfindungsgemäße Verfahren erforderlichen Eigenschaften : verbesserte Aktivität und Selektivität sowie weniger anfällig gegenüber möglicherweise bei der Reaktion anfallender Kontaktgifte. Vorteilhaft ist

weiterhin die große Sedimentationsgeschwindigkeit, wodurch der Katalysator zu der mehrfachen Wiederverwendung leicht abgetrennt werden kann.

Zur Durchführung des erfindungsgemäßen Verfahrens wird der umzusetzende Alkohol oder Aldehyd in einem heizbaren Rührbehälter, der ausgerüstet ist mit einem externen Gaskreislauf vorgelegt. Das System wird mit Wasserstoff gefüllt und auf die Reaktionstemperatur von 150-250 °C, vorzugsweise 170-230 °C, gebracht. Der Druck wird so gewählt, daß der eingesetzte Rohstoff nicht siedet. In der Regel wird die Reaktion von Alkoholen oder Aldehyden mit Siedepunkten > 150 °C bei Normaldruck oder geringfügig erhöhtem Druck, d. h. ca. 1,2 bis 2 bar, durchgeführt. Der aktivierte Katalysator mit einem Anteil von vorzugsweise 0,2-3 Gew.-%, bezogen auf eingesetzten Alkohol/Aldehyd, kann vor oder auch nach dem Aufheizen zugegeben werden. Die Reaktion wird durch Einleiten des Amins begonnen, wobei entstandenes Reaktionswasser durch Kondensation aus dem Kreislauf entfernt wird. Höhere Aminkonzentrationen im Kreislauf stören nicht, es wird jedoch vorzugsweise so gearbeitet, daß nur soviel Amin eingeleitet wird, wie direkt verbraucht werden kann. Das hat den Vorteil, daß in der Gesamtbilanz das Verhältnis von Amin zu Alkohol bzw. Aldehyd bei ca. 1,0 bis 1,1 eingestellt werden kann.

Ebenso unkritisch ist gegenüber den bekannten Verfahren die Menge des im Kreislauf geführten — gegebenenfalls Inertgase enthaltenden — Wasserstoffgases. Typisch sind hier Mengen von 20-300 l/kg Alkohol bzw. Aldehyd-Einsatzprodukt und Stunde. Nach Beendigung der Reaktion können Ausgangs- und Zwischenprodukte mittels Trägergasdestillation entfernt werden. Sie können in den Prozeß rückgeführt werden. Als Zwischenprodukt auftretende höher siedende sekundäre Amine, wie z. B. Didodecylamin, können nach Beendigung der Aminzugabe durch Zusatz von Alkohol, z. B. Methanol, in das gewünschte tertiäre Amin überführt werden. Dann wird das Reaktionsgemisch abgekühlt, der Katalysator wird durch einfache Filtration abgetrennt und kann erneut eingesetzt werden.

Der Katalysator kann sehr oft wiederverwendet werden, ohne daß nennenswerte Verminderungen bezüglich Umsetzungsgrad und Selektivität zu beobachten sind.

Die anfallenden Reaktionsprodukte enthalten bei Alkoholumsetzungsgraden von ca. 99 % störende Aminierungsnebenprodukte nur in Mengen von < 2 % und weisen damit einen Reinheitsgrad auf, der für die meisten Folgeprozesse wie z. B. die Herstellung quartärer Ammoniumverbindungen ausreicht, so daß eine Destillation sich in der Regel erübrigt.

Ein weiterer Vorteil des erfindungsgemäß verwendeten Katalysators ist dessen Eigenschaft, auch in Gegenwart von Wasserstoff die gegebenenfalls vorhandenen Mehrfachbindungen nicht zu reduzieren, so daß Produkte mit unverändert hohen Jodzahlen hergestellt werden können.

Katalysatorherstellung

Katalysator I

Zur Herstellung des Katalysators werden 200,1 g Kupfer-(II)-hydroxid und 156,8 g Zinn-(II)-oxid fein miteinander verrieben und in 1500 ml Toluol suspendiert (Verfahren A). Der Katalysator hat ein molares Kupfer : Zinn-Verhältnis von 1,76 (Katalysator I).

Katalysatoraktivierung

Zur Aktivierung des Katalysators wird die Suspension des Katalysators I auf 100 °C erwärmt und es werden unter gutem Rühren 314 g Diisobutylaluminiumhydrid (DIBAH) zugetropft. Die Zugabe wird so gesteuert, daß das Toluol leicht siedet. Nachdem alles DIBAH eingebracht ist, wird die Suspension 1 Stunde auf Siedetemperatur gehalten. Danach wird unter Rühren auf Raumtemperatur abgekühlt und der Rührer stillgelegt, so daß der feinverteilte Katalysator sedimentieren kann. Dann wird das überstehende Toluol abgezogen und es wird mit 500 ml Toluol aufgeschlämmt. Nach erneutem Sedimentieren wird wiederum mit 500 ml Toluol gewaschen und sedimentiert. Dieser Prozeß wird noch zweimal wiederholt. Im toluolfeuchten Zustand kann der aktive Katalysator ohne Schwierigkeiten gehandhabt werden.

Katalysator II

155,5 g Kupfer-(II)-hydroxid und 41,4 g Zinn-(II)-oxid werden wie in Beispiel 1 innig vermischt (Verfahren A). Sie werden wie in Beispiel 1 beschrieben mit 195,5 g DIBAH aktiviert. Das molare Kupfer : Zinn-Verhältnis beträgt 5,2.

Katalysator III

Zu einer 30 %igen Lösung von Kupfer-(II)-nitrat in Wasser werden 9,6 Gew.-% Zinn(II)-formiat gegeben. Aus dieser Lösung wird der Katalysatorvorläufer mit 30 % Kaliumcarbonatlösung bei 60° ausgefällt. Nach vollständiger Fällung wird abfiltriert und der Filterkuchen neutral und nitratfrei gewaschen. Anschließend wird bei 60° im Vakuum vollständig getrocknet (Verfahren B). 186 g dieses Katalysators werden, wie in Beispiel 1 beschrieben, mit 213 g DIBAH aktiviert. Das molare Kupfer : Zinn-Verhältnis beträgt 3,5.

Katalysator IV

155,5 g Kupfer(II)-hydroxid und 22,5 g Zinn-II-oxid werden innig vermischt (Verfahren A) und mit 181 g DIBAH, wie in Beispiel 1 beschrieben, aktiviert. Das molare Kupfer : Zinn-Verhältnis beträgt 9,55.

Katalysator V

Zu einer 30 %igen Lösung von Kupfer II-nitrat in Wasser werden, wie unter Katalysator III beschrieben, 13,9 Gew.-% Zinn-(II)-formiat gegeben (Verfahren B). Es wird, wie dort beschrieben, weiter aufgearbeitet. 245 g dieses Katalysators werden, wie in Beispiel 1 beschrieben, mit 325 g Triisobutylaluminium (TIBA) aktiviert. Das molare Kupfer : Zinn-Verhältnis beträgt 2,4.

Katalysator VI

200 g Kupfer-(II)-hydroxid und 157 g Zinn-II-oxid werden innig vermischt (Verfahren A) und, wie unter Beispiel 1 beschrieben, anstelle von DIBAH mit 680 g Butyloctylmagnesium (BOMAG) aktiviert. Das Kupfer : Zinn-Verhältnis beträgt 1,76.

Katalysator VII

15,5 g Kupfer-II-hydroxid und 138 g Zinn-(II)-oxid werden innig vermischt und mit 120 g DIBAH, wie unter Beispiel 1 beschrieben, aktiviert. Das Kupfer : Zinn-Verhältnis beträgt 0,16.

Katalysator VIII

5,0 kg Kupfer-II-hydroxid und 2,8 kg Zinn-II-oxid werden innig vermischt, in 60 l Toluol suspendiert und mit 7,05 kg DIBAH, wie unter Beispiel 1 beschrieben, aktiviert. Das molare Kupfer : Zinn-Verhältnis beträgt 2,47.

Katalysator IX

216 g Kupfer-(I)-Cyanid und 237 g Zinn-(II)-acetat werden wie in Beispiel 1 innig vermischt (Verfahren A) und in 1500 ml Pentran suspendiert. Zu dieser Suspension werden 700 g Triethylaluminium (TEA) bei Raumtemperatur unter gutem Rühren und Kühlung gegeben. Nachdem alles TEA eingebracht worden ist, wird 2 Stunden nachgerührt. Die weitere Behandlung zur Entfernung von nicht umgesetztem TEA wird, wie unter Katalysator I, Katalysatoraktivierung, beschrieben, durchgeführt.

Katalysator X

Entspricht dem Verfahren zur Herstellung von Katalysator VIII bis auf den Unterschied, daß die Aktivierung bei 140 Grad Celsius in Dekalin als inertem Lösungsmittel durchgeführt wird.

Katalysator XI

Der Katalysator des Beispiels VI wurde wie in Beispiel 1 beschrieben aktiviert. Als metallorganische Verbindungen wurden dabei verwendet :

XI a) Trimethylaluminium (TMA)
b) Di-i-octylaluminiumhydrid (TOAH)
c) Tri-n-dodecylaluminium (TDA)
d) Methyl-butylmagnesium (MeBuMg)
e) Di-n-octylmagnesium (Oc2Mg)

Diese Verbindungen wurden im Überschuß von 0,1 Äquivalenten, bezogen auf die reduzierbaren Gruppen im Katalysator, eingesetzt.

(Siehe Tabelle Seite 6 f.)

Tabelle - Katalysatorherstellung

| Katalysator | Herstellungsverfahren | Zusammensetzung Mol Cu/Mol Sn | Aktivator |
|---|---|---|---|
| I | A | 1,76 | DIBAH ① |
| II | A | 5,2 | DIBAH |
| III | B | 3,5 | DIBAH |
| IV | A | 9,55 | DIBAH |
| V | B | 2,4 | TIBA ② |
| VI | A | 1,76 | BOMAG③ * |
| VII | A | 0,16 | DIBAH |
| VIII | A | 2,47 | DIBAH |
| IX | A | 1,12 | TEA |
| X | A | 2,47 | DIBAH |

①DIBAH = Diisobutylaluminiumhydrid
②TIBA = Triisobutylaluminium
③BOMAG = Butyloctylmagnesium (* eingetragenes Warenzeichen der Schering AG)

Aminierung

Beispiel 1

In einem Rührwerk mit 3 Liter Volumen und Begasungrührer werden 700 g Laurylalkohol vorgelegt. Der Reaktor wird mit Wasserstoff gefüllt. Bei eingeschaltetem Rührer stellt sich ein Wasserstoffkreislauf von 71 l/h/kg Alkohol ein. Zusätzlich strömen 29 l $H_2$/h/kg Alkohol durch die Apparatur. Der Reaktorinhalt wird auf die Reaktionstemperatur von 180 °C gebracht. Ist die Temperatur erreicht, werden 13,5 g Katalysator I zugegeben und es wird mit Zugabe von Monomethylamin (MMA) begonnen. Das entstandene Reaktionswasser wird durch Kondensation aus dem Kreislaufgas entfernt. Nach 6,5 Stunden ist ein Alkoholumsatz von 99,8 % erreicht, der Gehalt an tertiärem Amin beträgt 85 Gew.-%, davon sind 95,6 Gew.-% Methyldidodecylamin. Der Katalysator wird durch Filtration abgetrennt.

Die folgenden Beispiele werden analog Beispiel 1 durchgeführt.

Beispiel 2 a

| | |
|---|---|
| Reaktorvolumen | 3 Liter |
| Laurylalkohol | 700 g |
| $H_2$-Kreislauf | 100 l/h/kg Alkohol |
| $H_2$-Zusatz | 29 l/h/kg Alkohol |
| Amin | Monomethylamin |
| Temperatur | 220 °C |
| Katalysator | 12,0 g Katalysator V |

Nach 4 Stunden beträgt der Alkoholumsatz 99,7 %, der Gehalt an tertiärem Amin beträgt 89,5 %, davon sind 96,8 Gew.-% Methyldidodecylamin.

Beispiel 2 b

Nach Ende der Reaktion nach Beispiel 2 a wird die Aminzufuhr eingestellt, der Wasserstoffkreislauf wird bei der Reaktionstemperatur von 220 °C aufrechterhalten. Unter diesen Bedingungen werden leichter flüchtige Bestandteile, wie sekundäres Methyldodecylamin, Dimethyldodecylamin, Restmengen Laurylalkohol und ein Teil der nicht Stickstoff enthaltenden Nebenprodukte abgetrieben und durch Kondensation aus dem Kreislauf entfernt. Der Gehalt an tertiären Aminen im Produkt steigt auf 91,1 Gew.-%, davon sind Methyldidodecylamin 97,9 Gew.-%.

Beispiel 2 c

Zu dem nach Beispiel 2 b hergestellten Produkt, das noch den Katalysator enthält, wird bei der Reaktionstemperatur von 220 °C Methanol in den Kreislauf eingespeist. Das im Produkt noch enthaltene

sekundäre Didodecylamin reagiert unter diesen Bedingungen weiter zu dem gewünschten Methyldidodecylamin. Der tertiäre Amingehalt steigt auf 93,7 Gew.-%, davon sind 98,0 Gew.-% Methyldidodecylamin.

Beispiel 3

| | |
|---|---|
| Reaktorvolumen | 30 Liter |
| Alkohol | 7 kg Laurylalkohol |
| $H_2$-Kreislauf | 500 l/h/kg Alkohol |
| $H_2$-Zusatz | 7 l/h/kg Alkohol |
| Amin | Dimethylamin (DMA) |
| Temperatur | 210 °C |
| Katalysator | 102,9 g Katalysator III |

Nach 11 Stunden beträgt der Alkoholumsatz 99,3 %, der Gehalt an tertiärem Amin beträgt 97,9 Gew.-%, davon sind 95,0 Gew.-% Dimethyldodecylamin.

Beispiel 4

| | |
|---|---|
| Reaktorvolumen | 3 Liter |
| Alkohol | 700 g Laurylalkohol |
| $H_2$-Kreislauf | 71 l/h/kg Alkohol |
| $H_2$-Zusatz | 29 l/h/kg Alkohol |
| Amin | Dimethylamin |
| Temperatur | 210 °C |
| Katalysator | 18,0 g Katalysator I |

Nach 9 Stunden beträgt der Alkoholumsatz 99,8 %, der Gehalt an tertiärem Amin beträgt 97,0 Gew.-%, davon sind 95,2 Gew.-% Dimethyldodecylamin.

Durch einfache Destillation wird Dimethyldodecylamin mit einer Reinheit von 98,7 % erhalten.

Vergleichsbeispiel 1

Der Versuch wird analog zu Beispiel 4 durchgeführt.

| | |
|---|---|
| Reaktorvolumen | 3 Liter |
| Alkohol | 700 g Laurylalkohol |
| $H_2$-Kreislauf | 67 l/h/kg Alkohol |
| $H_2$-Zusatz | 22 l/h/kg Alkohol |
| Amin | Dimethylamin |
| Temperatur | 210 °C |
| Katalysator | 9,3 Kupferchromit (Südchemie G99B) |

Nach 7 Stunden beträgt der Alkoholumsatz 93,9 %, der Gehalt an tertiärem Amin beträgt 75,3 Gew.-%, davon sind 94,6 Gew.-% Dimethyldodecylamin.

Vergleichsbeispiel 2

Der Versuch wird analog zu Beispiel 4 durchgeführt. Der Katalysator wird jedoch bereits bei Raumtemperatur zugefügt. Es wird im Wasserstoffstrom auf Reaktionstemperatur aufgeheizt.

| | |
|---|---|
| Reaktorvolumen | 3 Liter |
| Alkohol | 900 g Laurylalkohol |
| $H_2$-Kreislauf | 67 l/h/kg Alkohol |
| $H_2$-Zusatz | 22 l/h/kg Alkohol |
| Amin | Dimethylamin |
| Temperatur | 210 °C |
| Katalysator | 9,0 g $Cu(OH)_2$ + $Ni(OH)_2$ Cu/Ni = 1.72 |

Nach 4,3 Stunden beträgt der Alkoholumsatz 99,6 %, der Gehalt an tertiärem Amin beträgt 70,6 Gew.-%, davon sind 81,9 Gew.-% Dimethyldodecylamin.

Vergleichsbeispiel 3

Der Versuch wird analog zu Beispiel 4 durchgeführt. Der Katalysator wird erst bei Reaktionstemperatur hinzugefügt.

| | |
|---|---|
| Reaktorvolumen | 3 Liter |
| Alkohol | 900 g Laurylalkohol |
| $H_2$-Kreislauf | 67 l/h/kg Alkohol |
| $H_2$-Zusatz | 28 l/h/kg Alkohol |
| Amin | Dimethylamin |
| Temperatur | 210 °C |
| Katalysator | 9,0 g $Cu(OH)_2$ + $Ni(OH)_2$, Cu/Ni = 1.72 |

Nach 3 Stunden beträgt der Alkoholumsatz 99,8 %, der Gehalt an tertiärem Amin beträgt 93,0 Gew.-%, davon sind 82,3 Gew.-% Dimethyldodecylamin.

Vergleiche zu Beispiel 4

| Vergleich | Katalysator | Alkoholumsatz | Produktzusammensetzung tert.Amine | Me2NR | R2NMe | R3N |
|---|---|---|---|---|---|---|
| | | | | (Gewichtsprozent) | | |
| 1 | Cu/Cr | 94,6 | 75,3 | 94,6 | 2,7 | 2,7 |
| 2 | Cu/Ni | 99,6 | 70,6 | 81,9 | 16,9 | 1,2 |
| 3 | Cu/Ni | 99,8 | 93,0 | 82,3 | 16,5 | 1,2 |
| Beispiel | | | | | | |
| 4 | Cu/Sn | 99,8 | 97,0 | 95,2 | 4,5 | 0,3 |

Beispiel 5

| | |
|---|---|
| Reaktionsvolumen | 3 Liter |
| Alkohol | 700 g Laurylalkohol |
| $H_2$-Kreislauf | 71 l/h/kg Alkohol |
| $H_2$-Zusatz | 29 l/h/kg Alkohol |
| Amin | Dimethylamin |
| Temperatur | 210 °C |
| Katalysator | 12,0 g Katalysator III |

Nach 7 Stunden beträgt der Alkoholumsatz 99,5 %, der Gehalt an tertiärem Amin beträgt 96,1 Gew.-%, davon sind 93,0 Gew.-% Dimethyldodecylamin.

Beispiel 6

| | |
|---|---|
| Reaktionsvolumen | 3 Liter |
| Alkohol | 700 g Laurylalkohol |
| $H_2$-Kreislauf | 71 l/h/kg Alkohol |
| $H_2$-Zusatz | 29 l/h/kg Alkohol |
| Amin | Dimethylamin |
| Temperatur | 190 °C |
| Katalysator | 14,0 g Katalysator VI |

Nach 10 Stunden beträgt der Alkoholumsatz 99,7 %, der Gehalt an tertiärem Amin beträgt 95,0 Gew.-%, davon sind 97,1 Gew.-% Dimethyldodecylamin.

Beispiel 7

| | |
|---|---|
| Reaktorvolumen | 3 Liter |
| Alkohol | 700 g Laurylalkohol |
| $H_2$-Kreislauf | 70 l/h/kg Alkohol |
| $H_2$-Zusatz | 30 l/h/kg Alkohol |
| Amin | Dimethylamin |
| Temperatur | 190 °C |
| Katalysator | 12,0 g Katalysator IV |

Nach 7 Stunden beträgt der Alkoholumsatz 99,7 %, der Gehalt an tertiärem Amin beträgt 88,4 Gew.-%, davon sind 96,7 Gew.-% Dimethyldodecylamin.

Beispiel 8

| | |
|---|---|
| Reaktorvolumen | 3 Liter |
| Alkohol | 700 g Stearylalkohol |
| $H_2$-Kreislauf | 69 l/h/kg Alkohol |
| $H_2$-Zusatz | 30 l/h/kg Alkohol |
| Amin | Monomethylamin |
| Temperatur | 230 °C |
| Katalysator | 15,0 g Katalysator II |

Nach 6 Stunden beträgt der Alkoholumsatz 99,4 %, der Gehalt an tertiärem Amin beträgt 89,0 Gew.-%, davon sind 91,6 Gew.-% Distearylmethylamin.

Tabelle Aminierung

| Beispiel | Katalysator | Alkohol | Amin | Temperatur °C |
|---|---|---|---|---|
| 1 | I | Lauryl | MMA (1) | 180 |
| 2a,b,c | V | Lauryl | MMA | 220 |
| 3 | III | Lauryl | DMA (2) | 210 |
| 4 | I | Lauryl | DMA | 210 |
| 5 | III | Lauryl | DMA | 210 |
| 6 | VI | Lauryl | DMA | 190 |
| 7 | IV | Lauryl | DMA | 190 |
| 8 | II | Stearyl | MMA | 230 |

| Beispiel | Alkoh.-umsatz | Gehalt an tert.Aminen (Gew.-%) | Produktzusammensetzung der tertiären Amine | | |
|---|---|---|---|---|---|
| | | | $Me_2NR$ (3) (Gew.-%) | $R_2NMe$ (Gew.-%) | $R_3N$ (Gew.-%) |
| 1 | 99,8 | 85,0 | 2,5 | 95,6 | 1,9 |
| 2a | 99,7 | 89,5 | 2,0 | 96,8 | 1,2 |
| 2b | 99,7 | 91,1 | 0,9 | 97,9 | 1,2 |
| 2c | 99,7 | 93,7 | 0,8 | 98,0 | 1,2 |
| 3 | 99,3 | 97,9 | 95,0 | 4,8 | 0,2 |
| 4 | 99,8 | 97,0 | 95,2 | 4,5 | 0,3 |
| 5 | 99,5 | 96,1 | 93,0 | 6,8 | 0,2 |
| 6 | 99,7 | 95,0 | 97,1 | 2,1 | 0,8 |
| 7 | 99,7 | 88,4 | 96,7 | 2,8 | 0,5 |
| 8 | 99,4 | 89,0 | 7,4 | 91,6 | 1,0 |

(1) MMA : Monomethylamin
(2) DMA : Dimethylamin
(3) $Me_2NR$ : Me = Methylrest
R = Rest des Einsatzalkohols
N = Stickstoff

## Beispiel 9

| | |
|---|---|
| Reaktorvolumen | 300 Liter |
| Alkohol | 170 kg Dodecylalkohol |
| $H_2$-Kreislauf | 120 l/h/kg Alkohol |
| $H_2$-Zusatz | 0,1 l/h/kg Alkohol |
| Amin | Monomethylamin |
| Temperatur | 200 °C |
| Katalysator | 8,1 kg Katalysator VIII |

Die Reaktion wird in einem 300 l Rührwerk mit externem Gaskreislauf durchgeführt. Das Amin wird in nahezu stöchiometrischen Mengen zugegeben, d. h. es wird so zudosiert, daß die Aminkonzentration 0,5 Vol-% (Fall a-f) bzw. 1,5 Vol-% (Fall g-i) im Kreislaufgas nicht überschreitet. Zur Bestimmung des Amingehaltes wird ein geringer Gasstrom von 0,1 l/h/kg Alkohol dem Kreislauf entnommen und einer Infrarotmessung zugeführt. Dieser meßtechnisch bedingte geringe Gasverlust wird durch frischen Wasserstoff aufgefüllt. Nach beendeter Reaktion wird der Katalysator durch Sedimentation vom Produkt abgetrennt und ohne frischen Katalysatorzusatz wieder verwendet. Die Reaktion ist nach 8-9 h vollständig abgelaufen. Das Produkt ist farblos und frei von gelösten Metallspuren.

(Siehe Tabelle Seite 11f.)

## Beispiel 10

| | |
|---|---|
| Reaktorvolumen | 3 l |
| Alkohol | 500 g Isotridecylalkohol (Isomerengemisch |
| $H_2$-Kreislauf | — |
| $H_2$-Zusatz | 150 l/h/kg Alkohol |
| Amin | Monomethylamin |
| Temperatur | 210 °C |
| Katalysator | 23 g Katalysator VIII |

Die Reaktion wird in einem 3 l Rührwerk ohne Wasserstoffkreislauf durchgeführt. Die Aminkonzentration im Eintritt beträgt 6,25 Vol-%. Nach 6 Stunden wird die Reaktion beendet. Das Produkt enthält neben 14,6 Gew.-% des Alkoholisomerengemisches 84,6 Gew.-% des tertiären Dialkylmethylamins.

## Beispiel 11

| | |
|---|---|
| Reaktorvolumen | 3 l |
| Alkohol | 500 g Benzylalkohol |
| $H_2$-Kreislauf | 60 l/h/kg Alkohol |
| $H_2$-Zusatz | 60 l/h/kg Alkohol |
| Amin | Monomethylamin |
| Temperatur | 180°-210 °C |
| Katalysator | 20 g Katalysator VIII |

Die Reaktion wird in einem 3 l Rührwerk mit Gaskreislauf unter Normaldruck durchgeführt. Es wird mit einer Anfangstemperatur von 180 °C begonnen, da der Reaktorinhalt mit einsetzender Reaktion stark siedet und schäumt. Mit fortschreitender Reaktion wird eine Temperatur von 210 °C erreicht. Nach 8 Stunden beträgt der Alkoholumsatz 99,2 %. Das Produkt enthält neben 73,6 Gew.-% des Dibenzylmethylamins 15,5 Gew.-% Benzaldehyd.

## Beispiel 12

| | |
|---|---|
| Reaktorvolumen | 3 l |
| Einsatzgemisch | 500 g mit 36,6 Gew.-% Dodecylaldehyd<br>36,4 Gew.-% Tetradecylaldehyd<br>27,0 Gew.-% Tetradecylalkohol |
| $H_2$-Kreislauf | — |
| $H_2$-Zusatz | 150 l/h/kg Einsatzgemisch |
| Amin | Monomethylamin |
| Temperatur | 210 °C |
| Katalysator | 25 g Katalysator VIII |

Nach 6 Stunden ist das Einsatzgemisch zu 99,1 % umgesetzt. Das Produkt besteht zu 85,6 Gew.-% aus einem Gemisch von Dialkylmethylaminen mit den Kettenlängen $C_{12}$ und $C_{14}$.

| Beispiel | MMA im Kreislaufgas (Vol-%) | Alkohol Umsatz (%) | Gehalt an tert. Aminen (Gew.-%) | Produktzusammensetzung der tertiären Amine $R_2NMe$ (Gew.-%) | $RNMe_2$ (Gew.-%) | $R_3N$ (Gew.-%) |
|---|---|---|---|---|---|---|
| 9 a | 0,5 | 99,3 | 93,0 | 98,0 | 0,8 | 1,2 |
| b | 0,5 | 99,2 | 95,5 | 97,9 | 0,4 | 1,7 |
| c | 0,5 | 99,6 | 93,5 | 97,2 | 0,4 | 2,4 |
| d | 0,5 | 99,3 | 94,0 | 97,6 | 0,4 | 2,0 |
| e | 0,5 | 99,5 | 94,1 | 98,3 | 0,3 | 1,4 |
| f | 0,5 | 99,5 | 93,3 | 98,4 | 0,3 | 1,3 |
| g | 1,5 | 99,4 | 93,0 | 97,8 | 0,8 | 1,4 |
| h | 1,5 | 99,5 | 93,5 | 98,4 | 0,5 | 1,1 |
| i | 1,5 | 99,2 | 94,2 | 97,7 | 1,5 | 1,0 |

Beispiel 13

| | |
|---|---|
| Reaktorvolumen | 30 l |
| Alkohol | 12 kg Stearylalkohol |
| $H_2$-Kreislauf | 290 l/h/kg Alkohol |
| $H_2$-Zusatz | 1,2 l/h/kg Alkohol |
| Amin | Monomethylamin |
| Temperatur | 200 °C |
| Katalysator | 360 g Katalysator VIII |

Der Amingehalt im Kreislauf wird auf 1-2 Vol-% eingestellt. Nach 6 Stunden beträgt der Alkoholumsatz 99,1 %, der Gehalt an tertiärem Amin beträgt 97,6 % Gew.-%. Davon sind 95,4 Gew.-% Distearylmethylamin.

Beispiel 14

| | |
|---|---|
| Reaktorvolumen | 30 l |
| Alkohol | 12 kg Talgalkohol① (soft tallow) |
| $H_2$-Kreislauf | 200 l/h/kg Alkohol |
| $H_2$-Zusatz | 1,0 l/h/kg Alkohol |
| Amin | Monomethylamin |
| Temperatur | 200 °C |
| Katalysator | 360 g Katalysator VIII |

Der Amingehalt im Kreislauf wird auf 1-2 Vol-% eingestellt. Nach 5,5 Stunden beträgt der Alkoholumsatz 99,7 %, der Gehalt an tertiären Aminen beträgt 92,3 Gew.-%. Davon sind 91,6 Gew.-% Dialkylmethylamin.

①Talgalkohol ist ein technisches Gemisch aus $C_{16-18}$-Alkoholen mit geringen $C_{14}$-Anteilen, welcher auch noch ungesättigte Alkohole (Jodzahl 48) enthält.

Beispiel 15

| | |
|---|---|
| Reaktorvolumen | 30 l |
| Alkohol | 12 kg Oleylalkohol (Jz 85) |
| $H_2$-Kreislauf | 275 l/h/kg Alkohol |
| $H_2$-Zusatz | 1,1 l/h/kg Alkohol |
| Amin | Monomethylamin |
| Temperatur | 200 °C |
| Katalysator | 360 g Katalysator VIII |

Nach 5 Stunden beträgt der Alkoholumsatz 99,5 %, der Gehalt an tertiären Aminen beträgt 94,8 Gew.-%. Davon sind 92,5 Gew.-% Dioleylmethylamin.

Beispiel 16

| | |
|---|---|
| Reaktorvolumen | 3 l |
| Alkohol | 700 g Laurylalkohol |
| $H_2$-Kreislauf | — |
| $H_2$-Zusatz | 80 l/h/kg Alkohol |
| Amin | Ammoniak |
| Temperatur | 220 °C |
| Katalysator | 22 g Katalysator VIII |

Nach 5 Stunden beträgt der Alkoholumsatz 98 % ; der Gehalt an Aminen beträgt 93,6 Gew.-%, davon sind 27 Gew.-% Dilaurylamin.

Beispiel 17

| | |
|---|---|
| Reaktorvolumen | 3 l |
| Alkohol | 700 g Laurylalkohol |
| $H_2$-Kreislauf | 70 l/h/kg Alkohol |
| $H_2$-Zusatz | 30 l/h/kg Alkohol |
| Amin | n-Butylamin |
| Temperatur | 210 °C |
| Katalysator | 22 g Katalysator VIII |

Nach 5 Stunden beträgt der Alkoholumsatz 99,3 % ; der Gehalt an tertiären Aminen beträgt 92 Gew.-%, davon sind 96,9 Gew.-% Butyl-Dilaurylamin.

Beispiel 18

| | |
|---|---|
| Reaktorvolumen | 3 l |
| Alkohol | 700 g Hexanol-(1) |
| $H_2$-Kreislauf | 70 l/h/kg Alkohol |
| $H_2$-Zusatz | 30 l/h/kg Alkohol |
| Amin | Monomethylamin |
| Temperatur | 180 °C |
| Katalysator | 22 g Katalysator VIII |

Nach 8 Stunden beträgt der Alkoholumsatz 99,1 % ; der Gehalt an tertiären Aminen beträgt 90 Gew.-%, davon sind 96,5 Gew.-% Dihexylmethylamin.

Beispiel 19

| | |
|---|---|
| Reaktorvolumen | 3 l |
| Alkohol | 700 g Decylalkohol |
| $H_2$-Kreislauf | 70 l/h/kg Alkohol |
| $H_2$-Zusatz | 30 l/h/kg Alkohol |
| Amin | Dimethylamin |
| Temperatur | 210 °C |
| Katalysator | 22 g Katalysator VIII |

Nach 8 Stunden beträgt der Alkoholumsatz 99,5 % ; der Gehalt an tertiären Aminen beträgt 92,1 Gew.-%, davon sind 96,8 Gew.-% Decyldimethylamin.

Beispiel 20

In einem 3 Liter Rührwerk werden 537 g Oleylamin mit 256 g 2-Ethylhexanol in Gegenwart von 40 g Katalysator X bei 215 °C und 2,0 bar zur Reaktion gebracht. Das entstandene Reaktionswasser wird durch Kondensation aus dem Wasserstoffkreislauf von 20 l/h/kg Alkohol entfernt. Nach 9 Stunden ist die Reaktion beendet. Im Reaktionsprodukt sind 94 % Dioctyloleylamin enthalten.

Beispiel 21

In einem 3 Liter Rührwerk werden 540 g Stearylalkohol mit 257 g 2-Ethylhexylamin in Gegenwart von 40 g Katalysator X bei 215 °C und 2,0 bar zur Reaktion gebracht. Das entstandene Reaktionswasser wird durch Kondensation aus dem Wasserstoffkreislauf von 20 l/h/kg Alkohol entfernt. Nach 9 Stunden ist die Reaktion beendet. Im Reaktionsprodukt sind 95,2 Distearyl-2-ethyl-hexyl-amin enthalten.

Beispiel 22

Entsprechend Beispiel 6 wurden die Katalysatoren des Beispiels XI zur Aminierung eingesetzt. Es wurden die folgenden Ergebnisse erhalten :

| Katalysator | Alkohol-umsatz | Gehalt an tert. Aminen Gew.-% | Produktzusammensetzung der tertiären Amine | | |
|---|---|---|---|---|---|
| | | | Me2NR | R2NMe | R3N |
| XI a | 99,8 | 96,2 | 96,1 | 2,2 | 1,7 |
| b | 99,5 | 95,8 | 96,8 | 2,2 | 1,0 |
| c | 99,4 | 95,5 | 97,2 | 1,7 | 1,1 |
| d | 99,7 | 96,0 | 97,1 | 2,0 | 0,9 |
| e | 99,6 | 96,1 | 96,7 | 2,1 | 1,1 |

**Patentansprüche**

1. Verfahren zur Herstellung von Aminen durch Umsetzung von Alkoholen und/oder Aldehyden mit bis zu 26 Kohlenstoffatomen mit primären und/oder sekundären Aminen mit 1 bis 26 Kohlenstoffatomen oder Ammoniak in Gegenwart eines Katalysators bei erhöhter Temperatur und gegebenenfalls unter erhöhtem Druck in der Flüssigphase, dadurch gekennzeichnet, daß als Katalysator ein trägerfreier Katalysator aus einer Kombination von Kupfer- und Zinnverbindungen verwendet wird, der mit einem metallorganischen Reduktionsmittel aktiviert worden ist.
2. Verfahren gemäß Anspruch 1, dadurch gekennzeichnet, daß man zur Aktivierung insbesondere Diisobutylaluminiumhydrid, Butyloctylmagnesium, Triisobutylaluminium in mindestens äquivalenten Mengen, bezogen auf reduzierbare Gruppen im Katalysator, einsetzt.
3. Verfahren gemäß den Ansprüchen 1 und 2, dadurch gekennzeichnet, daß als Alkohole primäre Alkohole, die gegebenenfalls verzweigt oder ungesättigt sein können und 6 bis 22 Kohlenstoffatome in der Kette aufweisen, verwendet werden.
4. Verfahren gemäß Anspruch 3, dadurch gekennzeichnet, daß als Alkohole aliphatische Alkohole mit 10 bis 18 Kohlenstoffatomen in der Kette verwendet werden.
5. Verfahren gemäß den Ansprüchen 1 bis 4, dadurch gekennzeichnet, daß anstelle der Alkohole die korrespondierenden Aldehyde verwendet werden.
6. Verfahren gemäß den Ansprüchen 1 bis 5, dadurch gekennzeichnet, daß als Amine primäre oder sekundäre Amine oder deren Mischungen mit 1 bis 4 Kohlenstoffatomen in jedem Alkylrest verwendet werden.
7. Verfahren gemäß Anspruch 6, dadurch gekennzeichnet, daß als Amine Monomethylamin oder Dimethylamin verwendet werden.
8. Verfahren gemäß den Ansprüchen 1 bis 7, dadurch gekennzeichnet, daß als Katalysatoren die Oxide und/oder Hydroxide von Kupfer und Zinn in molaren Verhältnissen von 0,1 bis 10 verwendet werden.
9. Verfahren gemäß Anspruch 8, dadurch gekennzeichnet, daß das molare Verhältnis von Kupfer zu Zinn zwischen 1,5 bis 5,5 liegt.

**Claims**

1. Process for the preparation of amines by reacting alcohols and/or aldehydes having up to 26 carbon atoms with primary and/or secondary amines having from 1 to 26 carbon atoms or with ammonia in the presence of a catalyst at elevated temperature, and optionally under elevated pressure, in the liquid phase, characterised in that the catalyst used is an unsupported catalyst consisting of a combination of copper and tin compounds that has been activated by an organometal reducing agent.
2. Process according to claim 1, characterised in that there is used for the activation especially diisobutylaluminium hydride, butyloctylmagnesium or triisobutylaluminium in at least equivalent amounts based on reducible groups in the catalyst.
3. Process according to claims 1 and 2, characterised in that there are used as alcohols primary alcohols that may optionally be branched or unsaturated and that contain from 6 to 22 carbon atoms in the chain.
4. Process according to claim 3, characterised in that there are used as alcohols aliphatic alcohols having from 10 to 18 carbon atoms in the chain.
5. Process according to claims 1 to 4, characterised in that instead of the alcohols the corresponding aldehydes are used.
6. Process according to claims 1 to 5, characterised in that there are used as amines primary or secondary amines or mixtures thereof having from 1 to 4 carbon atoms in each alkyl radical.
7. Process according to claim 6, characterised in that there are used as amines monomethylamine or dimethylamine.
8. Process according to claims 1 to 7, characterised in that there are used as catalysts oxides and/or hydroxides of copper and tin in molar ratios of from 0.1 to 10.
9. Process according to claim 8, characterised in that the molar ratio of copper to tin is between 1.5 and 5.5.

**Revendications**

1. Procédé de préparation d'amines par réaction d'alcools et/ou d'aldéhydes ayant jusqu'à 26 atomes de carbone avec des amines primaires et/ou secondaires ayant de 1 à 26 atomes de carbone ou avec l'ammoniac, à chaud et le cas échéant sous pression, en phase liquide et en présence d'un catalyseur, procédé caractérisé en ce que le catalyseur est un catalyseur sans support formé d'une association de composés de cuivre et d'étain, qui a été activé avec un agent réducteur organométallique.
2. Procédé selon la revendication 1 caractérisé en ce que l'on effectue l'activation en particulier avec

l'hydrure de di-sobutylaluminium, le butyloctyl-magnésium ou le triisobutylaluminium en des proportions au moins équivalentes par rapport aux groupes réductibles du catalyseur.

3. Procédé selon la revendication 1 ou 2 caractérisé en ce que l'on utilise comme alcools des alcools primaires pouvant être éventuellement ramifiés ou insaturés et qui ont de 6 à 22 atomes de carbone.

4. Procédé selon la revendication 3 caractérisé en ce que l'on utilise des alcools aliphatiques ayant de 10 à 18 atomes de carbone.

5. Procédé selon l'une quelconque des revendications 1 à 4, caractérisé en ce que l'on utilise au lieu des alcools les aldéhydes correspondants.

6. Procédé selon l'une quelconque des revendications 1 à 5, caractérisé en ce que l'on utilise comme amines des amines primaires ou secondaires dont chacun des alkyles a de 1 à 4 atomes de carbone, ou des mélanges de ces amines.

7. Procédé selon la revendication 6 caractérisé en ce que l'on utilise la monométhylamine ou la diméthylamine.

8. Procédé selon l'une quelconque des revendications 1 à 7, caractérisé en ce que l'on utilise comme catalyseurs les oxydes et/ou hydroxydes de cuivre et d'étain dans un rapport molaire de 0,1 à 10.

9. Procédé selon la revendication 8 caractérisé en ce que le rapport molaire du cuivre à l'étain est compris entre 1,5 et 5,5.